# EUROPEAN PATENT APPLICATION

(11) **EP 1 790 719 A1**
(43) Date of publication of application: **30.05.2007**
(21) Application number: 05770350.6
(22) Date of filing: 09.08.2005
(51) Int. Cl.: C12N 15/09, C12Q 1/68, G01N 21/78

(54) **METHOD OF ASSAYING ALPHA 1,4-n-ACETYLGLUCOSAMINE TRANSFERASE (ALPHA 4GnT) mNRA**

(30) Priority: 19.08.2004 JP 2004239781
(71) Applicant: Tosoh Corporation, Syuunan-shi, Yamaguchi 746-8501 (JP)
(72) Inventor: SAITO, Juichi, 2420028 (JP); HAYASHI, Toshinori, 2280804 (JP)
(74) Representative: Pautex Schneider, Nicole Véronique
(86) International application number: PCT/JP2005/014888
(87) International publication number: WO 2006/019065

(57) **Abstract**

A method for assaying α1,4-N-acetylglucosamine transferase (α4GnT) mRNA present in a sample, the method comprising a step of using a first primer homologous to at least a portion downstream from the 5'-end of a specified nucleotide sequence of the RNA and a second primer complementary to at least a portion upstream from the 3'-end of the specified nucleotide sequence to produce double-stranded DNA containing the promoter sequence and the specified nucleotide sequence downstream from the promoter sequence, wherein at least one of the first and second primers has a promoter sequence at the 5'-end, a step of using the double-stranded DNA as template to produce an RNA transcript, a step of using the RNA transcript in turn as template for DNA synthesis to produce the double-stranded DNA, a step of nucleic acid amplification in which the aforementioned steps are repeated under conditions that simultaneously promote each of the steps, and a step of assaying the amount of the RNA transcript.

## Description

### Technical Field

The present invention relates to a method for rapid assay of α1,4-N-acetylglucosamine transferase (α4GnT) in a convenient, isothermal and single-stage manner. The invention belongs to the field of medicine and especially clinical diagnosis, and provides a useful index for early diagnosis of cancer, monitoring of treatment, judgment of prognosis and determination of treatment course.

### Background Art

α1,4-N-acetylglucosamine transferase (abbreviated as α4GnT) is an enzyme having activity that transfers an N-acetylglucosamine from an N-acetylglucosamine donor to a galactose residue present on the non-reducing terminal of an N-acetylglucosamine receptor sugar chain with an α1,4 bond that was found to be expressed in stomach tissue (see Nakayama, J. et al. (1999) Proc. Natl. Acad. Sci. USA, 96, 8991-8996; Zhang, M.X. et al., (2001) J. Histochem. Cytochem., 49, 587-596). In these documents, α4GnT is reported to be involved in the biosynthesis of a characteristic GlcNAcα1 → 4Galβ → R structure present in the O-glycan terminal of a glycoprotein secreted from mucosal cells. In addition, in these documents, said GlcNAcα1 → 4Galβ → R was found to be a tumor-associated sugar chain antigen of stomach cancer, and the aforementioned α4GnT was found to be expressed at a high frequency in stomach cancer cells.

Recently, increases in α4GnT messenger RNA (mRNA) levels have been observed in stomach cancer, pancreatic cancer, papillary cancer and bile duct cancer as a result of measuring α4GnT mRNA in a mononuclear cell fraction of peripheral blood using RT-PCR, and have also been indicated as allowing detection of early stage stomach cancer (see Japanese Unexamined Patent Publication (Kokai) No. 2001-46077; Shimizu, F., et al., (2003) Lab. Invest., 83, 187-197). Namely, the aforementioned findings suggest measurement of α4GnT mRNA to be effective for early diagnosis of stomach and other cancers, monitoring of therapeutic effects and predicting prognosis.

An example of a means for measuring α4GnT mRNA with high sensitivity is a method involving measurement of an amplification product by amplifying α4GnT mRNA by RT-PCR. In this case, however, two steps consisting of a reverse transcription (RT) step and a PCR step are typically required, and this not only causes the procedure to be complex while also worsening reproducibility, but it also increases the risk of secondary contamination. The RT and PCR steps combined normally take more than 2 hours, thus making this method unsuitable for processing large numbers of specimens or reducing testing costs.

Although real-time PCR (RT-PCR), which involves carrying out a PCR step in the presence of an intercalator fluorescent dye and measuring an increase in fluorescence, is typically used to quantify a target RNA, in this method, there is a problem that non-specific amplification products such as primer dimers may be detected. In addition, since RT-PCR also amplifies DNA, in the case of amplifying only mRNA, it is necessary to completely remove chromosomal DNA by DNase treatment and so forth in a nucleic acid extraction step, thereby leading to increased complexity of the nucleic acid extraction procedure. Moreover, since it is necessary to rapidly raise the reaction temperature in the case of PCR, this proves to be a hindrance in reducing power consumption and costs of the reaction apparatus during automation.

On the other hand, reported examples of methods for amplifying RNA only include the NASBA method (see Japanese Patent Nos. 2650159 and 3152927) and the TMA method (see Japanese Patent No. 3241717). These RNA amplification methods employ a chain reaction wherein a primer comprising the promoter sequence for the target RNA, reverse transcriptase and if necessary ribonuclease H (RNase H) are used for synthesis of double-stranded DNA containing the promoter sequence, RNA polymerase is used for synthesis of RNA containing the specified nucleotide sequence of the target RNA, and the RNA is in turn used as template for synthesis of double-stranded DNA containing the promoter sequence. After RNA amplification, the amplified RNA is detected by electrophoresis or a hybridization method using a nucleic acid probe bound to a detectable label.

As has been described above, although these RNA amplification methods are suited for simple mRNA measurement by amplifying only RNA at a constant temperature and in a single step, they have the problems of detection by hybridization and so forth requiring a complex procedure while also preventing quantification with good reproducibility.

As convenient methods for amplification and assay of mRNA there may be mentioned the method described by Ishiguro et al. (Japanese Unexamined Patent Publication (Kokai) No. 2000-14400 and Ishiguro, T., et al., (2003) Anal. Biochem., 314, 77-86). In this method, RNA amplification is carried out in the presence of a nucleic acid probe which is labeled with an intercalating fluorescent dye and is designed so that when it forms a complementary double strand with the target nucleic acid, the intercalating fluorescent dye portion undergoes a change in fluorescent property by intercalating into the complementary double strand, and the change in fluorescent property is measured, whereby it is possible to simultaneously accomplish RNA amplification and assay in a convenient, isothermal and single-stage manner in a sealed vessel.

### Disclosure of the Invention

The assay of α4GnT mRNA is useful for early diagnosis of stomach cancer, pancreatic cancer and other cancers, but using RT-PCR requires a two-stage process with a complicated procedure and necessitating abrupt increase/decrease in the reaction temperature; this leads to a risk of secondary contamination and poor reproducibility, while constituting an obstacle to development of a convenient and automated assay. The present invention overcomes the aforementioned problems by providing a method for assaying α4GnT mRNA in a convenient, rapid, isothermal and single-stage manner.

As a result of much diligent research directed toward solving the aforementioned problems, the present inventors have constructed a method for assaying α4GnT mRNA in a convenient, rapid, isothermal and single-stage manner which applies the RNA amplification method explained above. Specifically, by measuring the level of amplified RNA product obtained by an RNA amplification process wherein a first primer and second primer (at least one of which has a promoter sequence at the 5' end) are used to produce double-stranded DNA containing the promoter sequence, the double-stranded DNA is used as template to produce an RNA transcript, and the RNA transcript in turn is used as template for DNA synthesis to produce the double-stranded DNA, it has become possible to assay α4GnT mRNA in a convenient, isothermal and single-stage manner.

### Brief Explanation of the Drawings

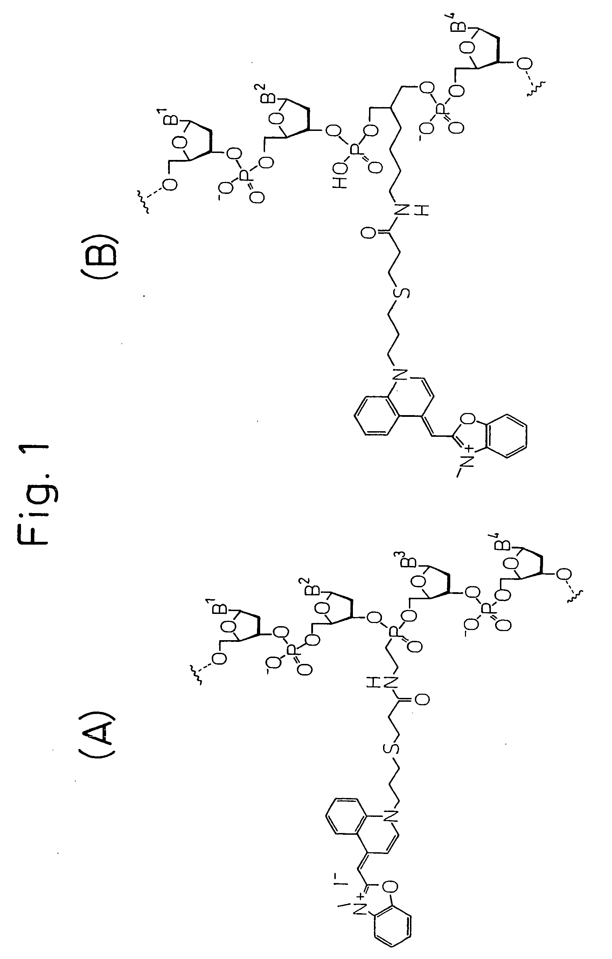

Fig. 1 shows the structure of the intercalating fluorescent dye-labeled nucleic acid probe prepared in Example 2. B¹, B², B³ and B⁴ represent bases.
   A) A probe comprising an intercalating fluorescent dye (oxazole yellow) bonded to the phosphate diester portion via a linker, according to the method of Ishiguro et al. (see Ishiguro, T., et al., (1996) Nucleic Acids Res., 24, 4992-4997). In order to prevent 3'-terminal - OH group extension reaction, the 3'-terminal -OH group was modified with glycolic acid. B) A probe comprising oxazole yellow bonded according to the method of Ishiguro et al. (see Ishiguro et al. (1996)), with an amino group introduced using commercially available Label-ON Reagents (Clontech). Here, the amino group was introduced at the nucleoside-lacking portion at the site of introduction (B³ in the drawing). In order to prevent 3'-terminal -OH group extension reaction, the 3'-terminal -OH group was modified with biotin.

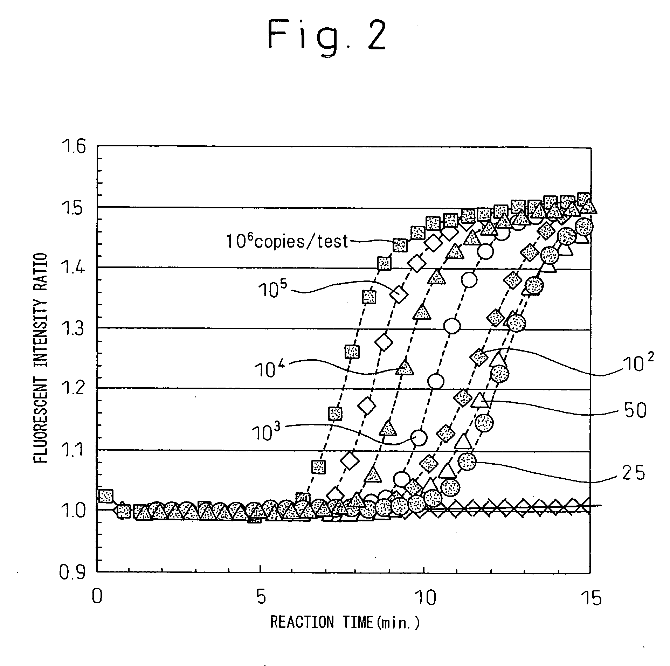

Fig. 2 shows fluorescence profiles obtained as a result of the measurement of Example 3.
   Shown are the results of carrying out RNA amplification according to the present invention simultaneously with periodic measurement of fluorescent intensity (excitation wavelength: 470 nm, fluorescent wavelength: 520 nm). The horizontal axis represents reaction time, and the vertical axis represents fluorescent intensity ratio (fluorescent intensity of reaction mixture/background fluorescence). The numbers of copies in the figure represent the initial numbers of copies of α4GnT Bl RNA (comprising base numbers 7 to 1242) used for one test (calculated by absorbance at 260 nm).

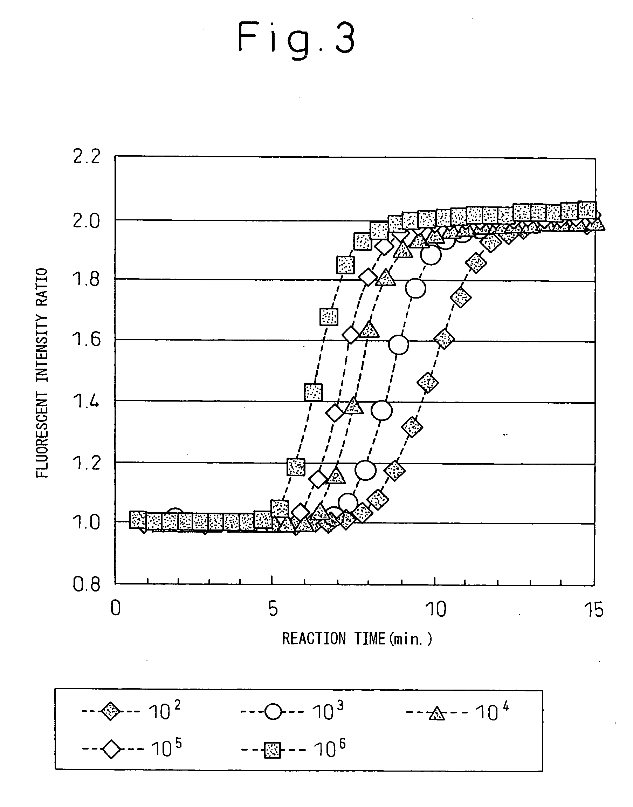

Fig. 3 shows fluorescence profiles obtained as a result of the measurement of Example 4.
   Shown are the results of carrying out RNA amplification according to the present invention simultaneously with periodic measurement of fluorescent intensity (excitation wavelength: 470 nm, fluorescent wavelength: 520 nm). The horizontal axis represents reaction time, and the vertical axis represents fluorescent intensity ratio (fluorescent intensity of reaction mixture/background fluorescence). The numerals in the legend of the figure represent the initial numbers of copies of α4GnT RNA (comprising base numbers 7 to 1242) used for one test (calculated by absorbance at 260 nm).

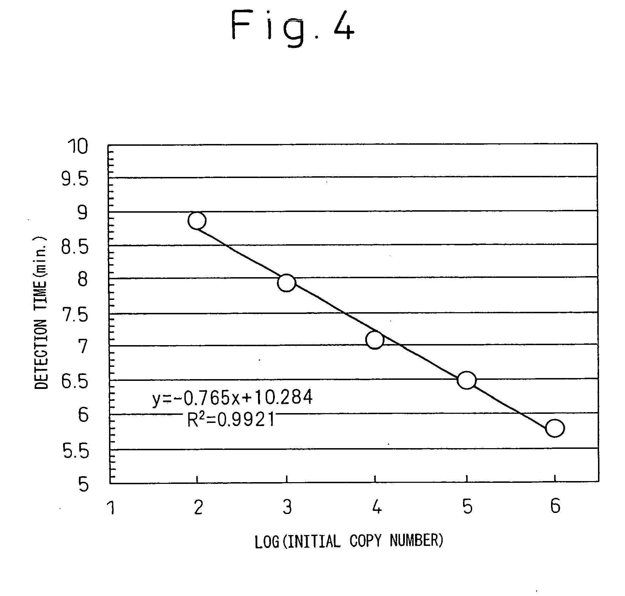

Fig. 4 is a calibration curve obtained from the results of Fig. 3. With the detection time defined as time at which the fluorescent intensity ratio reached 1.2 in the results of Fig. 3, the detection time was plotted with respect to the logarithm of initial number of copies of standard RNA. The equations in the drawing indicate a linear regression equation and correlation coefficient. The initial number of copies of unknown sample is calculated from this calibration curve and the detection time of the unknown sample obtained by the present method.

The following provides a detailed explanation of the present invention.

### Best Mode for Carrying Out the Invention

The invention is a method for assaying α4GnT mRNA present in a sample, the method comprising a step of using a first primer homologous to at least a portion downstream from the 5' end of a specified nucleotide sequence of the RNA and a second primer complementary to at least a portion upstream from the 3' end of the specified nucleotide sequence to produce a double-stranded DNA containing the promoter sequence and the specified nucleotide sequence downstream from the promoter sequence, wherein at least one of the first and second primers has a promoter sequence at the 5' end, a step of using the double-stranded DNA as template to produce an RNA transcript, a step of using the RNA transcript in turn as template for DNA synthesis to produce the double-stranded DNA, a step of nucleic acid amplification in which the aforementioned steps are repeated under conditions that simultaneously promote each of the steps, and a step of assaying the amount of the RNA transcript.

A sample according to the invention consists of nucleic acid extracted by a known method from a specimen such as blood, serum, plasma, tissue or lavage suspected of containing cancer cells.

A specified nucleotide sequence according to the present invention consists of at least a partial sequence of α4GnT mRNA or the sequence complementary thereto, and it is the sequence of the region defined between the first primer and second primer. According to the present invention, the RNA transcript from the specified nucleotide sequence is amplified. When the promoter sequence is added to the first primer, the α4GnT mRNA is preferably cleaved at the 5'-end of the specific nucleic acid sequence before serving as the template for cDNA synthesis. The cleavage method is not particularly restricted, but it is preferably a method using an enzyme with RNase H activity to cleave the RNA portion of an RNA-DNA hybrid formed by adding an oligonucleotide (a cleavage oligonucleotide) having a sequence complementary to the region overlapping and adjacent to the 5'-end of the specific nucleotide sequence of α4GnT mRNA, and preferably the cleavage oligonucleotide used has its 3'-terminal -OH appropriately modified, for example, aminated, to prevent extension reaction.

The target nucleic acid according to the present invention has a region in the specific base sequence that is not homologous or complementary to the first and second primers, while having a sequence that allows complementary binding with the intercalating fluorescent dye-labeled nucleic acid probe. Thus, the intercalating fluorescent dye-labeled nucleic acid probe is a sequence complementary to a portion of the specific nucleotide sequence according to the invention. According to one mode of the present invention, therefore, when the specified nucleotide sequence is a sequence homologous to α4GnT mRNA, the intercalating fluorescent dye-labeled nucleic acid probe has a sequence containing at least 15 contiguous bases of the sequence listed as SEQ ID NO: 5, and when the specified nucleotide sequence is a sequence complementary to α4GnT mRNA, the intercalating fluorescent dye-labeled nucleic acid probe has a sequence containing at least 15 contiguous bases of a sequence complementary to the sequence listed as SEQ ID NO: 5.

At least one of the first and second primers according to the present invention has a promoter sequence at the 5'-end. Namely, although it is sufficient for at least one of the first and second primers to have a promoter sequence, both the first and second primers may have a promoter sequence on the 5'-terminals thereof. In addition, the first primer is an oligonucleotide sufficiently complementary to the complementary sequence of α4GnT mRNA, while the second primer is an oligonucleotide sufficiently complementary to α4GnT mRNA. "Sufficient complementarity" means that complementary binding can occur with the specified nucleotide sequence or the sequence complementary to that sequence, under the reaction conditions (reaction temperature and composition of salts, etc.) for the nucleic acid amplification step of the invention. Such reaction conditions may be, for example, hybridization conditions at 43°C in the presence of 60 mM Tris, 17 mM magnesium chloride, 100 mM potassium chloride and 1 mM DTT.

In order to provide sufficient complementarity to a complementary sequence for α4GnT mRNA for the first primer, to α4GnT mRNA for the second primer and to the target nucleic acid for the intercalating fluorescent dye-labeled nucleic acid probe, the first primer preferably comprises at least 15 and more preferably at least 20 contiguous bases of the sequence listed as SEQ ID NO: 1 or 3, the second primer preferably comprises at least 15 and more preferably at least 20 contiguous bases of the sequence listed as SEQ ID NO: 2 or 4, and the intercalating fluorescent dye-labeled nucleic acid probe preferably comprises at least 15 and more preferably at least 20 contiguous bases of the sequence listed as SEQ ID NO: 5 or 6 or the sequence complementary to that sequence.

A combination of sequences in which the first primer preferably comprises at least 15 contiguous nucleotides, and more preferably at least 20 contiguous nucleotides of the sequence listed as SEQ ID NO. 1, the second primer preferably comprises at least 15 contiguous nucleotides, and more preferably comprises 20 contiguous nucleotides, of the sequence listed as SEQ ID NO. 2, and the intercalator fluorescent dye-labeled nucleic acid probe preferably comprises at least 15 contiguous nucleotides, and more preferably at least 20 contiguous nucleotides, of the sequence listed as SEQ ID NO. 5 or the complementary sequence of said sequence, or a combination in which the first primer preferably comprises at least 15 contiguous nucleotides, and more preferably at least 20 contiguous nucleotides of the sequence listed as SEQ ID NO. 3, the second primer preferably comprises at least 15 contiguous nucleotides, and more preferably comprises 20 contiguous nucleotides, of the sequence listed as SEQ ID NO. 4, and the intercalator fluorescent dye-labeled nucleic acid probe preferably comprises at least 15 contiguous nucleotides, and more preferably at least 20 contiguous nucleotides, of the sequence listed as SEQ ID NO. 6 or the complementary sequence of said sequence, is preferable. Although these combinations are more preferable aspects of the positional relationship of sequences complementarily binding with α4GnT mRNA, other combinations can also be used amplify and measure α4GnT mRNA.

Alternatively, the first primer, second primer and intercalating fluorescent dye-labeled nucleic acid probe may each have a nucleotide sequence that hybridizes with the complementary strand having the sequence listed as SEQ ID NO. 1 or 3, SEQ ID NO. 2 or 4 and SEQ ID NO. 5 or 6, respectively, under highly stringent conditions, for example, under the aforementioned reaction conditions for the nucleic acid amplification step of the invention.

The promoter sequence used for the present invention is a sequence to which RNA polymerase binds to initiate transcription, and specific sequences corresponding to different RNA polymerases are known. There are no particular restrictions on the RNA polymerase, but common ones such as T7 phage RNA polymerase, T3 phage RNA polymerase and SP6 phage RNA polymerase are preferred, and their corresponding promoter sequences may be used.

The method for assaying α4GnT mRNA according to the present invention requires certain enzymes (an enzyme having RNA-dependent DNA polymerase activity for single-stranded RNA template (reverse transcriptase), an enzyme having RNase H activity, an enzyme having DNA-dependent DNA polymerase activity for single-stranded DNA template, and an enzyme having RNA polymerase activity). Any of these enzymes may be enzymes having different activities, or a plurality of enzymes having each activity may be used. For example, an enzyme having RNA polymerase activity may be added to a reverse transcriptase having RNA-dependent DNA polymerase activity for single-stranded RNA template, RNase H activity and DNA-dependent DNA polymerase activity for single-stranded DNA template, or if necessary an enzyme with RNase H activity may be further added as a supplement. Preferred as the reverse transcriptase are AMV reverse transcriptase, M-MLV reverse transcriptase and their derivatives, from the standpoint of flexible utility.

When the reverse transcription reaction is carried out in the presence of the first primer and the second primer, the second primer binds to the specified base sequence of α4GnT mRNA and carries out cDNA synthesis with the enzyme having RNA-dependent DNA polymerase activity. The RNA portion of the obtained RNA-DNA hybrid is degraded by the enzyme having RNase H activity, and dissociates so that the first primer may bind to the cDNA.

Next, double-stranded DNA derived from the specified base sequence and containing the promoter sequence at the 5'-end is produced by the enzyme having DNA-dependent DNA polymerase activity. The double-stranded DNA contains the specified base sequence downstream from the promoter sequence, and an RNA transcript derived from the specified base sequence is produced by the enzyme having RNA polymerase activity. The RNA transcript serves as template for the double-stranded DNA synthesis by the first and second primers, so that a series of reactions occur as a chain reaction and result in amplification of the RNA transcript.

In order to promote the chain reaction, it is of course necessary to add at least buffer solution, magnesium salt, potassium salt, nucleoside triphosphates and ribonucleoside triphosphates as known elements essential for each of the enzymes. In addition, additives such as dimethylsulfoxide (DMSO), dithiothreitol (DTT), bovine serum albumin (BSA), sugars and the like may be added to control the reaction efficiency.

For example, when using AMV reverse transcriptase and T7 RNA polymerase, the reaction temperature is preferably set in a range of 35°C-65°C, and most preferably it is set in a range of 40°C-44°C. The RNA amplification step proceeds isothermally, and the reaction temperature may be set to any desired temperature at which the reverse transcriptase and RNA polymerase exhibit their activity.

The amount of amplified RNA transcript can be measured by a known nucleic acid assay method. As such assay methods, there may be used methods employing electrophoresis or liquid chromatography, or hybridization methods employing nucleic acid probes labeled with detectable labels. But these procedures involve multiple steps, and because the amplification product is removed out of the system for analysis, there is a high risk of escape of the amplification product into the environment as a cause of secondary contamination. To overcome these problems it is preferred to use a nucleic acid probe designed so that its fluorescent property changes upon complementary binding to the target nucleic acid. As a more preferred method, there may be mentioned a method wherein the nucleic acid amplification step is carried out in the presence of a nucleic acid probe which is labeled with an intercalating fluorescent dye and is designed so that when it forms a complementary double strand with the target nucleic acid, the intercalating fluorescent dye portion undergoes a change in fluorescent property by intercalating into the complementary double strand, and the change in fluorescent property is measured (see Japanese Unexamined Patent Publication (Kokai) No. 2000-14400 and Ishiguro, T., et al., (2003) op. cit.).

There are no particular restrictions on the intercalating fluorescent dye, and there may be used common dyes such as oxazole yellow, thiazole orange, ethidium bromide and their derivatives. The change in fluorescent property may be a change in fluorescent intensity. For example, it is known that in the case of oxazole yellow, intercalation into double-stranded DNA causes a notable increase in fluorescence at 510 nm (excitation wavelength of 490 nm). The intercalating fluorescent dye-labeled nucleic acid probe is an oligonucleotide with sufficient complementarity to the RNA transcript, and it has a structure with the intercalating fluorescent dye bonded to an end, a phosphate diester portion or a base portion via an appropriate linker, and with the 3'-terminal -OH group suitably modified to prevent extension from the 3'-terminal -OH (see Japanese Unexamined Patent Publication (Kokai) No. 8-211050; and, Ishiguro, T. et al., (1996) op. cit.). ).

Labeling of the oligonucleotide with the intercalating fluorescent dye may be accomplished by introducing a functional group into the oligonucleotide by a known method and bonding the intercalating fluorescent dye thereto (see Japanese Unexamined Patent Publication (Kokai) No. 2001-13147 and Ishiguro, T., et al., (1996) op. cit.). The method of introducing the functional group may employ the commonly used Label-ON Reagents (Clontech Laboratories, Inc.).

As one mode of the present invention, there are added to the sample an assay reagent containing at least the first primer (comprising at least 15 contiguous bases of the sequence listed as SEQ ID NO: 1) having the T7 promoter sequence at the 5' end, the second primer (comprising at least 15 contiguous bases of the sequence listed as SEQ ID NO: 2), an intercalating fluorescent dye-labeled nucleic acid probe (comprising at least 15 contiguous bases of the sequence listed as SEQ ID NO: 5), a cleavage oligonucleotide (comprising at least 15 bases of a sequence listed as SEQ ID NO: 22, and having a sequence complementary to the region overlapping and adjacent to the 5'-end of the specified base sequence), AMV reverse transcriptase, T7 RNA polymerase, a buffering agent, a magnesium salt, a potassium salt, nucleoside triphosphates, ribonucleoside triphosphates and dimethylsulfoxide (DMSO), and reaction is carried out at a constant reaction temperature of 35-65°C (preferably 40-44°C) while periodically measuring the fluorescent intensity of the reaction solution.

As another mode of the present invention, there are added to the sample an assay reagent containing at least the first primer (comprising at least 15 contiguous bases of the sequence listed as SEQ ID NO: 3) having the T7 promoter sequence at the 5' end, the second primer (comprising at least 15 contiguous bases of the sequence listed as SEQ ID NO: 4), an intercalating fluorescent dye-labeled nucleic acid probe (comprising at least 15 contiguous bases of the sequence listed as SEQ ID NO: 6), a cleavage oligonucleotide (comprising at least 15 bases of a sequence selected from the the group consisting of SEQ ID NOs: 23 to 25, and having a sequence complementary to the region overlapping and adjacent to the 5'-end of the specified base sequence), AMV reverse transcriptase, T7 RNA polymerase, a buffering agent, a magnesium salt, a potassium salt, nucleoside triphosphates, ribonucleoside triphosphates and dimethylsulfoxide (DMSO), and reaction is carried out at a constant reaction temperature of 35-65°C (preferably 40-44°C) while periodically measuring the fluorescent intensity of the reaction solution.

In each of the above mentioned mode, the fluorescent intensity results as an increase curve from the initial RNA amount, and therefore by drawing a calibration curve using standard RNA of known concentration it is possible to quantify the initial amount of RNA in an unknown sample. Moreover, since the fluorescent intensity is periodically measured, the measurement may be concluded at any desired point at which a significant increase in fluorescence is detected, and measurement results can be obtained usually within an hour, and within 30 minutes with an optimal system.

It is especially notable that all of the test materials in the assay reagent can be included in the same vessel. That is, the simple procedure of dispensing prescribed amounts of test materials into a single vessel will allow automatic amplification and detection of α4GnT mRNA to be conducted thereafter. The vessel may be constructed of, for example, a partially transparent material to allow external measurement of the signal emitted by the fluorescent dye, and a vessel that can be sealed after dispensing the test materials is particularly preferred to prevent contamination.

The RNA amplification and assay method according to the modes described above can be carried out in a single-stage and isothermal manner, and it is therefore more convenient than RT-PCR and is suitable for automation. However, because the reaction is carried out at a relatively low constant temperature of 35-65°C without denaturation and annealing as in RT-PCR, it is prone to non-specific amplification products such as primer dimers and to effects of the higher order structure of the RNA to be assayed, and therefore a much more elaborate design is necessary to construct the assay system compared to RT-PCR, for which reason it had not been previously possible to assay α4GnT mRNA in a rapid, convenient, isothermal and single-stage manner using the aforementioned RNA amplification and assay method. The present invention made it possible for the first time to assay α4GnT mRNA with high specificity and high sensitivity in a rapid, convenient, isothermal and single-stage manner.

The present invention can be applied to early diagnosis of stomach cancer, pancreatic cancer and other cancers, monitoring of therapeutic efficacy of chemotherapy and so on, micrometastasis, prognosis and as an indicator for determining a course of treatment.

### Examples

The present invention will now be further explained by examples, with the understanding that the invention is not limited by the examples.

### Example 1

α4GnT mRNA was prepared by carrying out in vitro transcription using as template a double-strand DNA having α4GnT cDNA (comprising nucleotide Nos. 7 to 1242; nucleotide sequence in accordance with the National Center Biotechnology Information Accession No. AF141315) downstream from an SP6 phage RNA polymerase promoter, and then completely digesting the double-strand DNA by treating with DNase I followed by purifying the RNA. Said RNA was quantified by measuring absorbance at 260 nm.

Although this RNA was measured in the following examples, this RNA can be adequately applied to measuring the α4GnT mRNA measured in the present invention.

### Example 2

An oligonucleotide probe labeled with an intercalator fluorescent dye was prepared. An amino group was inserted at the location of the 13th nucleotide from the 5'-terminal of the sequences described in SEQ ID NO. 18, 19 and 21 (A in SEQ ID NO. 18, C in SEQ ID NO. 19, and G in SEQ ID NO. 21) using Label-ON Reagents (Clontech), followed by modifying the 3'-terminal with biotin. Oxazole yellow was bound to the amino acid according to the method described in Ishiguro, T. et al., (1996) op. cit. (Fig. 1B). In addition, an oxazole yellow-labeled nucleic acid probe was prepared according to the method of Ishiguro, T. et al., (1996) op. cit. in which oxazole yellow was bound to a phosphate diester portion between the 9th C and the 10th T from the 5'-terminal of the sequence described in SEQ ID NO. 20 via a linker (Fig. 1A).

### Example 3

α4GnT mRNA having various numbers of initial copies was detected using the method of the present invention.
(1) The aforementioned α4GnT mRNA (comprising nucleotide Nos. 7 to 1242) was respectively diluted to 25, 50, 100 and 1000 copies/5 µl using an RNA diluent (10 mM Tris·HCl (pH 8.0), 1 mM EDTA, 0.25 U/µl ribonuclease inhibitor, 5 mM DTT) and used as RNA samples. The RNA diluent was used as a negative standard (0 copies).
(2) 20 µl of a reaction solution having the composition shown below were dispensed into 0.5 ml volume PCR tubes followed by the addition of 5 µl of the RNA samples described above thereto.
   Reaction solution composition: Concentrations indicate the final concentrations after addition of enzyme solution (30 µl)
   60 mM Tris·HCl (pH 8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO. 7): The first primer had the T7 polymerase promoter sequence (SEQ ID NO: 26) added to the 5'-end of the nucleotide sequence listed as this SEQ ID NO.
   1 µM second primer (SEQ ID NO. 8)
   25 nM intercalator fluorescent dye-labeled nucleic acid probe (SEQ ID NO. 19): Prepared using Label-ON Reagents used in Example 2
   0.16 µM cleavage oligonucleotide (SEQ ID NO. 22): 3'-terminal OH group of said oligonucleotide modified with an amino group
   6 U/30 µl ribonuclease inhibitor (Takara Bio)
   13% DMSO
(3) After warming the above reaction solution for 5 minutes at 43°C, 5 µl of enzyme solution having the composition shown below warmed for 2 minutes at 43°C were added.
   Enzyme solution composition: Final concentration during reaction (30 µl)
   2% sorbitol
   8 U/30 µl AMV reverse transcriptase (Takara Bio)
   142 U/30 µl T7 RNA polymerase (Gibco)
   3.6 µg/30 µl bovine serum albumin
(4) Next, a fluorescent spectrophotometer equipped with a heat regulating function and allowing direct measurement of the PCR tube was used for reaction at 43°C while periodically measuring the fluorescent intensity of the reaction mixture (excitation wavelength: 470 nm, fluorescent wavelength: 520 nm).

The time-related change in the fluorescent intensity ratio of the reaction mixture (fluorescent intensity value at prescribed time ÷ background fluorescent intensity value) is shown in Fig. 2, where the point of enzyme addition is defined as 0 minutes. The results in Fig. 2 show a fluorescent profile dependent on the initial concentration of α4GnT mRNA, whereby 25 copies/test of α4GnT mRNA could be detected within about 12 minutes. This indicates that α4GnT mRNA can be assayed at high sensitivity and high speed by the method of the invention.

### Example 4

α4GnT mRNA was measured according to the method of the present invention using various combinations of the first primers, second primers, intercalator fluorescent dye-labeled nucleic acid probes and cleavage oligonucleotides.
(1) The aforementioned α4GnT mRNA (comprising nucleotide Nos. 7 to 1242) was diluted to 10³ or 10² copies/5 µl using an RNA diluent (10 mM Tris·HCl (pH 8.0), 1 mM EDTA, 0.25 U/µl ribonuclease inhibitor, 5.0 mM DTT), and used as RNA samples.
(2) 20 µl of a reaction solution having the composition shown below was dispensed into 0.5 ml volume PCR tubes (Gene Amp Thin-Walled Reaction Tubes, Perkin-Elmer) followed by the addition of 5 µl of the above reaction solutions.
   Reaction solution composition: Concentrations indicate the final concentrations after addition of enzyme solution (30 µl)
   60 mM Tris·HCl (pH 8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO. shown in Table 1): The first primer comprises the T7 polymerase promoter sequence (SEQ ID NO: 26) added to the 5'-end of the nucleotide sequence listed as this SEQ ID NO.
   1 µM second primer (SEQ ID NO. shown in Table 1)
   25 nM intercalator fluorescent dye-labeled nucleic acid probe (SEQ ID NO. shown in Table 1)
   0.16 µM cleavage oligonucleotide (SEQ ID NO. shown in Table 1): 3'-terminal OH of said oligonucleotide modified with an amino group
   6 U/30 µl ribonuclease inhibitor (Takara Bio)
   13% DMSO
(3) The reaction solution was warmed for 5 minutes at 43°C followed by the addition of 5 µl of an enzyme solution having the composition shown below warmed for 2 minutes at 43°C.
   Enzyme solution composition: Final concentration during reaction (in 30 µl)
   2% sorbitol
   8 U/30 µl AMV reverse transcriptase (Takara Bio)
   142 U/30 µl T7 RNA polymerase (Gibco)
   3.6 µg/30 µl bovine serum albumin
(4) Next, a fluorescent spectrophotometer equipped with a heat regulating function and allowing direct measurement of the PCR tube was used for reaction at 43°C while periodically measuring the fluorescent intensity of the reaction mixture (excitation wavelength: 470 nm, fluorescent wavelength: 520 nm).

Table 1 shows the results, where (+) indicates that the fluorescent intensity ratio of the reaction mixture exceeded 1.2, and the time at that point is listed as the detection time, with the point of enzyme addition defined as 0 minutes.

In the case of using the combinations of the first primer, second primer, intercalator fluorescent dye-labeled nucleic acid probe and cleavage oligonucleotide shown in Table 1, 10³ copies/test of α4GnT RNA were detected within 20 minutes. Namely, α4GnT RNA was able to be rapidly detected either in the case of using a combination of the sequence listed as SEQ ID NO. 7 for the first primer, a sequence selected from SEQ ID NOs. 8 to 10 for the second primer, a sequence selected from SEQ ID NOs. 18 to 20 for the intercalator fluorescent dye-labeled nucleic acid probe, and the sequence listed as SEQ ID NO. 22 for the cleavage oligonucleotide, or in the case of using a combination of a sequence selected from SEQ ID NOs. 11 to 13 for the first primer, a sequence selected from SEQ ID NOs. 14 to 17 for the second primer, the sequence listed as SEQ ID NO. 21 for the intercalator fluorescent dye-labeled nucleic acid probe, and a sequence selected from SEQ ID NOs. 23 to 25 for the cleavage oligonucleotide. Here, SEQ ID NO. 7 is a partial sequence of SEQ ID NO. 1, each of SEQ ID NO. 8 to 10 is a partial sequence of SEQ ID NO. 2, each of SEQ ID NO. 18 to 20 is a partial sequence of SEQ ID NO. 5, each of SEQ ID NO. 11 to 13 is a partial sequence of SEQ ID NO. 3, each of SEQ ID NO. 14 to 17 is a partial sequence of SEQ ID NO. 4, and the sequence listed as SEQ ID NO. 21 is a partial sequence of SEQ ID NO. 6. On the basis of these results, α4GnT RNA was demonstrated to be able to be rapidly detected by RNA amplification and measurement using the first primer, second primer and intercalator fluorescent dye-labeled nucleic acid probe described in the present invention.

**Table 1 Assay Results Using Various Combinations of Oligonucleotides**

| Oligonucleotide Combination | | | | Assay Results | |
|---|---|---|---|---|---|
| First primer (SEQ ID NO.) | Second primer (SEQ ID NO.) | Intercalator fluorescent dye-labeled nucleic acid probe (SEQ ID NO.) | Cleavage oligonucleotide (SEQ ID NO.) | Detection time (min) | Judgment |
| 7 | 8 | 18 | 22 | 17.5 | + |
| 7 | 8 | 19 | 22 | 9.5 | + |
| 7 | 8 | 20 | 22 | 7.8 | + |
| 7 | 9 | 18 | 22 | 11.8 | + |
| 7 | 9 | 19 | 22 | 11.6 | + |
| 7 | 10 | 19 | 22 | 13.0 | + |
| 11 | 14 | 21 | 23 | 14.1 | + |
| 11 | 15 | 21 | 23 | 15.9 | + |
| 12 | 14 | 21 | 24 | 13.4 | + |
| 12 | 16 | 21 | 24 | 16.9 | + |
| 13 | 14 | 21 | 25 | 19.9 | + |
| 13 | 15 | 21 | 25 | 17.4 | + |
| 13 | 16 | 21 | 25 | 14.7 | + |
| 13 | 17 | 21 | 25 | 11.6 | + |

RNA amplification and fluorescence measurement were carried out using the combinations of oligonucleotide shown in the table above and using 10³ copies/test of α4GnT RNA for the sample. Samples with a fluorescent intensity ratio exceeding 1.2 were indicated as (+), and the time at that point was recorded as the detection time.

### Example 5

Quantification of α4GnT RNA was carried out using the method of the present invention.
(1) The aforementioned α4GnT RNA (comprising nucleotide nos. 7 to 1242) was diluted to 10², 10³, 10⁴, 10⁵ or 10⁶ copies/5 µl using an RNA diluent (10 mM Tris·HCl (pH 8.0), 1 mM EDTA, 0.25 U/µl ribonuclease inhibitor, 5.0 mM DTT) and used as standard RNA for a calibration curve. The diluent was used as a negative standard (NEG). Samples A (10² copies/5 µl), B (10³ copies/5 µl), C (10⁴ copies/5 µl), D (10⁵ copies/5 µl) and E (10⁶ copies/5 µl) were similarly prepared.
(2) 20 µl of a reaction solution having the composition shown below were dispensed into 0.5 ml volume PCR tubes (Gene Amp Thin-Walled Reaction Tubes, Perkin-Elmer) followed by the addition of 5 µl of the standard RNA and each sample thereto.
   Reaction solution composition: Concentrations indicate the final concentrations after addition of enzyme solution (30 µl)
   60 mM Tris·HCl (pH 8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO. 7): The first primer comprises the T7 polymerase promoter sequence (SEQ ID NO: 26) added to the 5'-end of the nucleotide sequence listed as this SEQ ID NO.
   1 µM second primer (SEQ ID NO. 8)
   25 nM intercalator fluorescent dye-labeled nucleic acid probe (SEQ ID NO. 20): Said nucleic acid probe was bound with oxazole yellow via a linker to phosphate diester in Example 2
   0.16 µM cleavage oligonucleotide (SEQ ID NO. 22): 3'-terminal OH group of said oligonucleotide modified with an amino group
   6 U/30 µl ribonuclease inhibitor (Takara Bio) 13% DMSO
(3) After warming the above reaction solution for 5 minutes at 43°C, 5 µl of enzyme solution having the composition shown below warmed for 2 minutes at 43°C were added.
   Enzyme solution composition: Final concentration during reaction (30 µl)
   2% sorbitol
   8 U/30 µl AMV reverse transcriptase (Takara Bio)
   142 U/30 µl T7 RNA polymerase (Gibco)
   3.6 µg/30 µl bovine serum albumin
(4) Next, a fluorescent spectrophotometer equipped with a heat regulating function and allowing direct measurement of the PCR tube was used for reaction at 43°C while periodically measuring the fluorescent intensity of the reaction mixture (excitation wavelength: 470 nm, fluorescent wavelength: 520 nm).

The time-related change in the fluorescent intensity ratio of the reaction mixture (fluorescent intensity value at prescribed time ÷ background fluorescent intensity value) is shown in Fig. 3, where the point of enzyme addition is defined as 0 minutes.

With the detection time defined as the time at which the fluorescent intensity ratio reached 1.2 based on the results in Fig. 3, a calibration curve was drawn from the detection time and logarithm of initial number of copies, as shown in Fig. 4. Moreover, the results of quantifying the number of copies of samples A, B, C, D and E from said calibration curve are shown in Table 3.

According to the results of Fig. 4, 10² copies/test were detected in about 10 minutes, and a favorable correlation was obtained between detection time and the logarithmic value of the initial number of copies. In addition, in the results of Table 3, values were obtained for the quantification results of samples A, B, C, D and E that corresponded to the amount of α4GnT RNA added. On the basis of these results, α4GnT RNA was demonstrated to be able to be quantified rapidly, specifically and with high sensitivity by the method of the present invention.

**Table 2 Quantification Results of α4GnT RNA**

| Sample | Assay Results | |
|---|---|---|
| | Detection Time (min) | No. of copies/test |
| A | 9.09 | 5.8 x 10 |
| B | 7.85 | 1.3 x 10³ |
| C | 7.00 | 1.4 x 10⁴ |
| D | 6.48 | 7.7 x 10⁴ |
| E | 5.90 | 6.6 x 10⁵ |

Results of calculating number of copies based on detection time obtained from the fluorescent profile for the measurement results for samples A (10² copies/5 µl), B (10³ copies/5 µl), C (10⁴ copies/5 µl), D (10⁵ copies/5 µl) and E (10⁶ copies/5 µl)

According to the present invention, α4GnT can be measured easily, rapidly and with high sensitivity at a constant temperature and in a single step. Thus, the present invention can be applied to early diagnosis of stomach cancer, pancreatic cancer and other cancers, and is useful for monitoring the effects of chemotherapy and other treatment, predicting prognosis and as an indicator for determining a course of treatment. Since the present invention can be carried out in a single step and is a sealed container, the risk of environmental contamination due amplification products causing secondary contamination can be minimized. In addition, since the present invention can be carried out easily and rapidly in a single step, it is able to process a large number of specimens even when using a manual technique, and is able to minimize the number of procedures, which is a factor causing poor reproducibility. Moreover, since the RNA amplification method of the present invention amplifies only RNA, mRNA can be precisely amplified and measured without a step involving complete removal of double-strand DNA in the manner of RT-PCR. Namely, the method of the present invention is optimally suited for highly sensitive and rapid expression analysis. In addition, since the method of the present invention can be carried out at a constant temperature and in a single step, it is not necessary to provide a thermal cycling mechanism in the manner of PCR, thereby facilitating automation.

## Claims

1. A method for assaying α1,4-N-acetylglucosamine transferase (α4GnT) mRNA present in a sample, the method comprising
(1) a step of using a first primer homologous to at least a portion downstream from the 5' end of a specified nucleotide sequence of said RNA and a second primer complementary to at least a portion upstream from the 3' end of said specified nucleotide sequence to produce a double-stranded DNA containing a promoter sequence and the specified nucleotide sequence downstream from the promoter sequence, wherein at least one of the first and second primers has the promoter sequence at the 5' end,
(2) a step of using the double-stranded DNA as template to produce an RNA transcript,
(3) a step of using the RNA transcript in turn as template for DNA synthesis for amplification of the RNA transcript in a chain reaction, and
(4) a step of assaying the amount of the RNA transcript.

2. A method for assaying α4GnT mRNA according to claim 1, **characterized by** use of a combination in which the first primer comprises at least 15 contiguous nucleotides of the sequence listed as SEQ ID NO. 1 and the second primer comprises at least 15 nucleotides of the sequence listed as SEQ ID NO. 2; or a combination in which the first primer comprises at least 15 contiguous nucleotides of the sequence listed as SEQ ID NO. 3 and the second primer comprises at least 15 nucleotides of the sequence listed as SEQ ID NO. 4.

3. A method for assaying α4GnT mRNA according to claim 1, **characterized in that** the assay of the amount of the RNA transcript is accomplished by measuring the change in the fluorescent property of a nucleic acid probe that is labeled with an intercalating dye and is designed so that when it forms a complementary double strand with the target nucleic acid, the intercalating fluorescent dye portion undergoes a change in fluorescent property by intercalating into the complementary double strand.

4. A method for assaying α4GnT mRNA according to claim 3, **characterized by** use of a combination in which the first primer comprises at least 15 contiguous nucleotides of the sequence listed as SEQ ID NO. 1, the second primer comprises at least 15 nucleotides of the sequence listed as SEQ ID NO. 2, and the intercalator fluorescent dye-labeled nucleic acid probe comprises at least 15 nucleotides of the sequence listed as SEQ ID NO. 5 or the complementary sequence of said sequence; or a combination in which the first primer comprises at least 15 contiguous nucleotides of the sequence listed as SEQ ID NO. 3, the second primer comprises at least 15 nucleotides of the sequence listed as SEQ ID NO. 4, and the intercalator fluorescent dye-labeled nucleic acid probe comprises at least 15 nucleotides of the sequence listed as SEQ ID NO. 6 or the complementary sequence of said sequence.

5. A reagent for assaying α4GnT mRNA comprising a combination of oligonucleotides in which a first primer comprises at least 15 contiguous nucleotides of the sequence listed as SEQ ID NO. 1 and a second primer comprises at least 15 contiguous nucleotides of the sequence listed as SEQ ID NO. 2; or a combination of oligonucleotides in which a first primer comprises at least 15 contiguous nucleotides of the sequence listed as SEQ ID NO. 3 and a second primer comprises at least 15 contiguous nucleotides of the sequence listed as SEQ ID NO. 4; wherein at least one of the first primer and the second primer has a promoter sequence on the 5'-terminal thereof.

6. A reagent for assaying α4GnT mRNA comprising a combination of oligonucleotides in which a first primer comprises at least 15 contiguous nucleotides of the sequence listed as SEQ ID NO.1, a second primer comprises at least 15 nucleotides of the sequence listed as SEQ ID NO. 2, and an intercalator fluorescent dye-labeled nucleic acid probe comprises at least 15 nucleotides of the sequence listed as SEQ ID NO. 5 or a complementary sequence of said sequence; or a combination of oligonucleotides in which a first primer comprises at least 15 contiguous nucleotides of the sequence listed as SEQ ID NO. 3, a second primer comprises at least 15 nucleotides of the sequence listed as SEQ ID NO. 4, and an intercalator fluorescent dye-labeled nucleic acid probe comprises at least nucleotides of the sequence listed as SEQ ID NO. 6 or a complementary sequence of said sequence; wherein at least one of the first primer and the second primer has a promoter sequence on the 5'-terminal thereof.

7. An oligonucleotide specific for α4GnT mRNA or a complementary strand thereof comprising: at least 15 contiguous nucleotides of any of the nucleotide sequences listed as SEQ ID NO. 1 to 6, or the complementary sequence of said sequences.


SEQUENCE LISTING

<110>  TOSOH Corporation

<120>  Method for Assay of α-1,4-N-Acetylglucosamine
Transferase (α-4GnT) mRNA

<130>  R779

<150>  JP2004-239781
<151>  2004-08-19

<160>  26

<170>  PatentIn version 3.3

<210>  1
<211>  45
<212>  DNA
<213>  Artificial

<220>
<223>  First Primer

<400>  1
ggcttcctct accagttcac cctgaagtcc agctgcctct tctgt                45

<210>  2
<211>  72
<212>  DNA

<213>  Artificial

<220>
<223>  Second Primer

<400>  2
cactcaggat aaatcttggc agcagactct acggaacagg agaccaaatg gggtggctcc     60

attctctctg ag                                                         72

<210>  3
<211>  84
<212>  DNA
<213>  Artificial

<220>
<223>  First Primer

<400>  3
atgaaaaggc tgcttgaaga cacaccattg ttttcatggt acaatcaaat caacgccagc     60

gcagagagaa actggctcca catc                                            84

<210>  4
<211>  87
<212>  DNA
<213>  Artificial

<220>
<223>  Second Primer

<400> 4

tccattacta gagtaccgag aagcctgcgc agccaaaaag ttctcctcag ggatgggcct 60

gatggagatg acatcggtgt ccatgta 87

<210> 5

<211> 58

<212> DNA

<213> Artificial

<220>

<223> Intercalator Fluorescene Dye Labelled Nucleic Acid
Probe

<400> 5

gccacgtctg tggctcagga gggcttccag cccctggtgg gacttgaaag aaggcaaa 58

<210> 6

<211> 45

<212> DNA

<213> Artificial

<220>

<223> Intercalator Fluorescene Dye Labelled Nucleic Acid
Probe

<400> 6

gccaccgtat ttccagatga tggccaggcg ggatgcatcc gagct 45

<210>  7
<211>  23
<212>  DNA
<213>  Artificial

<220>
<223>  First Primer

<400>  7
accctgaagt ccagctgcct ctt                                              23


<210>  8
<211>  24
<212>  DNA
<213>  Artificial

<220>
<223>  First Primer

<400>  8
aaatggggtg gctccattct ctct                                             24


<210>  9
<211>  24
<212>  DNA
<213>  Artificial

<220>
<223>  Second Primer

<400>  9
cagactctac ggaacaggag acca                                              24

<210>  10
<211>  24
<212>  DNA
<213>  Artificial

<220>
<223>  Second Primer

<400>  10
cactcaggat aaatcttggc agca                                              24

<210>  11
<211>  24
<212>  DNA
<213>  Artificial

<220>
<223>  First Primer

<400>  11
atgaaaaggc tgcttgaaga caca                                              24

<210>  12
<211>  23
<212>  DNA

<213>  Artificial

<220>
<223>  First Primer

<400>  12
cattgttttc atggtacaat caa                                              23

<210>  13
<211>  23
<212>  DNA
<213>  Artificial

<220>
<223>  First Primer

<400>  13
gcagagagaa actggctcca cat                                              23

<210>  14
<211>  24
<212>  DNA
<213>  Artificial

<220>
<223>  Second Primer

<400>  14
ggagatgaca tcggtgtcca tgta                                             24

<210>  15
<211>  24
<212>  DNA
<213>  Artificial

<220>
<223>  Second Primer

<400>  15
agttctcctc agggatgggc ctga                                              24

<210>  16
<211>  24
<212>  DNA
<213>  Artificial

<220>
<223>  Second Primer

<400>  16
cctgcgcagc caaaaagttc tcct                                              24

<210>  17
<211>  24
<212>  DNA
<213>  Artificial

<220>
<223>  Second Primer

<400>  17
cattactaga gtaccgagaa gcct                                              24

<210>  18
<211>  24
<212>  DNA
<213>  Artificial

<220>
<223>  Intercalator Fluorescene Dye Labelled Nucleic Acid
Probe

<400>  18
tggtgggact tgaaagaagg caaa                                              24

<210>  19
<211>  24
<212>  DNA
<213>  Artificial

<220>
<223>  Intercalator Fluorescene Dye Labelled Nucleic Acid
Probe

<400>  19
ccacgtctgt ggctcaggag ggct                                              24

<210>  20

<211>  20

<212>  DNA

<213>  Artificial

<220>

<223>  Intercalator Fluorescene Dye Labelled Nucleic Acid

Probe

<400>  20

gtctgtggct caggagggct                                                    20

<210>  21

<211>  24

<212>  DNA

<213>  Artificial

<220>

<223>  Intercalator Fluorescene Dye Labelled Nucleic Acid

Probe

<400>  21

tttccagatg atggccaggc ggga                                               24

<210>  22

<211>  24

<212>  DNA

<213>  Artificial

<220>

<223>  Cleaving Oligonucleotide

<400>  22
cagggtgaac tggtagagga agcc                                              24

<210>  23
<211>  24
<212>  DNA
<213>  Artificial

<220>
<223>  Cleaving Oligonucleotide

<400>  23
tttcatatcc aaagggaaga ggaa                                              24

<210>  24
<211>  24
<212>  DNA
<213>  Artificial

<220>
<223>  Cleaving Oligonucleotide

<400>  24
caatggtgtg tcttcaagca gcct                                              24

<210>  25
<211>  24
<212>  DNA

<213>  Artificial

<220>
<223>  Cleaving Oligonucleotide

<400>  25
ctctgcgctg gcgttgattt gatt                                              24

<210>  26
<211>  28
<212>  DNA
<213>  Artificial

<220>
<223>  T7 Polymerase Promoter Sequence

<400>  26
aattctaata cgactcacta tagggaga                                          28